# EUROPEAN PATENT SPECIFICATION

(11) **EP 2 946 803 B1**
(45) Date of publication and mention of the grant of the patent: **03.07.2019**
(21) Application number: 14169282.2
(22) Date of filing: 21.05.2014
(51) Int. Cl.: A61M 25/00

(54) **Reinforced urinary catheter**
Verstärkter Harnkatheter
Cathéter urinaire renforcé

(43) Date of publication of application: 25.11.2015
(73) Proprietor: Dentsply IH AB, 431 21 Mölndal (SE)
(72) Inventor: Dvärsäter, Gudmund, 431 39 Mölndal (SE); Schmid, Andrea, 435 42 Mölnlycke (SE); Davidsson, Fredrik, 431 67 Mölndal (SE)
(74) Representative: AWA Sweden AB

(56) References cited:
- WO-A1-2012/176189
- DE-U1-202005 009 946
- US-A1- 2009 071 851
- US-A1- 2012 203 210
- US-A1- 2012 271 282

## Description

### Field of the invention

The present invention relates to a urinary catheter assembly, comprising a reinforced catheter.

### Background of the invention

The present invention relates to a urinary catheter assembly, and in particular for urinary hydrophilic catheters. Catheters are commonly used for draining bodily fluids, e.g. from the bladder. In order to maintain the catheter in a clean and preferably sterile condition, each catheter is normally pre-packed in a receptacle by the manufacturer, thereby providing a catheter assembly. Urinary catheters are e.g. used by a large group of persons for intermittent catheterization, which is a daily-life procedure, taking place several times a day. Typically catheters for intermittent catheterization are used by patients suffering from urinary incontinence or by disabled individuals like para- or tetraplegics. Using an intermittent catheter, the bladder may be drained through a natural or artificial urinary canal. Many catheters for intermittent catheterization are provided with a hydrophilic coating or the like, providing a smooth and slippery surface when wetted, for safe and comfortable insertion in the urinary canal.

Many hydrophilic catheter assemblies include a supply of wetting fluid, either in direct contact with the catheter or in a separate compartment, for clean and convenient activation of the hydrophilic surface before use.

However, there is still a need for improved packages for such catheter assemblies. The package should preferably be relatively simple and cost-efficient to produce. Further, the package should be easy to handle, even for users with reduced dexterity. Still further, the package should enable adequate wetting of the catheter, and handling of the package in a clean manner. The package should also preferably be rather small, and discreet, with minimized volume and weight, so that it can easily be carried around by the user in his/hers daily life. Patent application US 2011/056852 by the same applicant discloses a urinary catheter assembly having a resealable opening. However, even though this catheter assembly is highly useable for short catheters, for female users, re-insertion of longer catheters, typically for male users, may be cumbersome and with the risk of spillage and the like. Male catheters may be 40 cm long, or even longer, and insertion of the tip portion through the resealable opening without touching the insertable part of the catheter is rather difficult, especially for users with reduced dexterity. Further, even though this product is small when used for short female catheters, it inevitably becomes much longer when used for long male catheters. Further, closing of the resealable opening after re-insertion of the catheter in a sufficiently sealed manner may also be difficult with this known catheter assembly, and again, especially for users with reduced dexterity.

Since a catheter needs to be of sufficient length to extend through the urethra, for instance having an insertable length of at least 200-350 mm for male users, the catheter assembly generally requires more space than is convenient. The assembly may thus be cumbersome to store, transport and handle, which is inconvenient not least for the individuals for whom catheterization is a daily-life procedure. To alleviate the inconvenience, there is a strive for less space consuming catheter assemblies which improve life quality for the user of catheters in that the assemblies can be handled and stored more discreetly, for instance in the pocket of a users clothing. Various attempts to produce more compact catheter assemblies have been made.

For example, US 2009/163884 by the same applicant discloses a catheter assembly including a urine collection bag integrated in a rearward part of the package. This assembly is made more compact by folding the rearward part over an elongate pocket housing the catheter. However, even though this solution makes the catheter assembly significantly more compact, the size of the product is still at least as long as the catheter itself.

There have also been attempts to make the catheter telescopic, but for natural reasons, such solutions are difficult and costly to produce.

Still further, it has been proposed to arrange the catheter in a curved disposition. For example, EP 2 106 821 by the same applicant discloses a catheter assembly in which the package comprises pleated regions allowing the package, and the catheter within it, to be arranged in a folded or double folded disposition. This solution greatly reduces the size of the catheter assembly. However, this type of package is relatively complicated and costly to produce. Still further, even though the curving of the catheter is arranged to exceed a minimum diameter of curvature, there is still a risk that the catheter will remain in a curved disposition even when withdrawn from the package. This is due to the fact that catheters are normally of a material having certain rigidity / shape memory characteristics. Storing the catheter assembly for a long period of time in a non-straight disposition, may result in kinking and distortion of the catheter, and imply resistance of easily reverting the catheter to its straight disposition prior to an intended use. This may be referred to as "permanent set" of the material, i.e. a permanent deformation remaining after a stress is released. The user may subsequently experience discomfort during insertion of the catheter if the insertable part remains in a non-straight disposition, and may also experience problems in handling and inserting the catheter properly. Further examples of known urinary catheter assemblies in which the catheters are arranged in a curved/curled state are presented in US 2012/0271282, disclosing a urinary catheter assembly according to the preamble of claim 1, and in US 2009/0071851. These assemblies face the same or similar problems as the ones discussed above. WO 2012/176189 discloses a reinforced catheter, the reinforcement being provided to increase pushability of the catheter, thereby avoiding kink during insertion of the catheter into the urethra.

Consequently, there is a need for a leaner and less bulky catheter assembly, and/or a catheter assembly which is less expensive to produce, meanwhile easy and convenient to handle and store, and in which the catheter will assume a relatively straight disposition before use. Especially there is a need for a catheter assembly which in an improved manner allows the catheter to, during storage, be curved such that minimum space consumption is required, and at the same time assuring that kinking and other deformation of the catheter is avoided.

### Summary of the invention

It is therefore an object of the present invention to provide a urinary catheter assembly, which at least alleviate the above-discussed problems of the previously known solutions.

This object is achieved with a urinary catheter assembly according to the appended claims.

According to the present invention, there is provided a urinary catheter assembly comprising:
a urinary catheter having a tubular shaft extending between a insertable end and a discharge end, wherein said tubular shaft is provided with an elastic reinforcement element having an extension in the axial direction of the tubular shaft; and
a receptacle arranged to accommodate and maintain the urinary catheter in a curled storage state, in which the tubular shaft of the catheter is arranged in at least one coil;
wherein said reinforcement element is arranged to provide a force to uncurl the catheter when removed from said receptacle.

The urinary catheter assembly of the invention can be made very compact, since the catheter is arranged in a curled, rolled-up state. A very long catheter, such as a male catheter, e.g. being about 40 cm long, can then be arranged in a package having a longest extension being lower than 10 cm. For example, the package may have a circular shape, and have a diameter of 7 or 8 cm. Such a compact package can easily be carried in a trouser pocket, a jacket pocket, a small handbag and the like. Thus, the urinary catheter assembly could easily be carried around without being noticed.

Further, the previously experienced drawbacks related to catheters stored in a rolled/curled state, such as shape deformation and kinking, are alleviated by means of the reinforcement. Hereby, the catheters tubes can be made in a conventional way, using well-known materials etc, and be provided with additional resilience by means of the reinforcement element. It has been surprisingly been found that by inclusion of an elastic reinforcement element, the catheters will reassume a straight or substantially straight state even after long time storage in a curled disposition, such as storage for 2-3 years.

Still further, provision of a reinforcement element in the catheter tube can be made easy and cost-efficiently, thereby not adding much to the manufacturing costs compared to conventional catheters. Since the reinforcement element may add extra strength to the catheter tube, the tube could even be made of less costly materials, thereby making production of the catheter even more cost-efficient.

Tubes having reinforcement elements are per se previously known. However, in these known solutions, the reinforcement elements are provided to enhance the stability of the tubes, e.g. to facilitate insertion into a body cavity. The reinforcement elements are not provided to improve resumption of a linear shape after having been stored in a curved or curled state for a long time. Further, most of these tubes are not catheters, and in particular not urinary catheters. Examples of such known reinforced tubes are e.g. found in US 6 148 818, US 5 454 061, WO 01/04530, US 5 906 036 and US 5 558 737. However, despite the difference in purpose and use of said reinforcement elements, similar reinforcement elements may be employed in the catheters of the present invention, and further the method of producing such reinforced catheters may be similar. Thus, the disclosures of said documents are hereby incorporated in their entirety by reference.

The urinary catheter is preferably a urinary catheter for intermittent, short time use. The term "short term use" indicates a use that is limited in time, and in particular limited to a time period of less than 15 minutes, and preferably less than 10 minutes, and most preferably less than 5 minutes.

The receptacle is preferably arranged to accommodate and maintain the urinary catheter in a curled storage state, in which the tubular shaft of the catheter is arranged in at least two coils, and preferably at least three coils. Hereby, an even more compact catheter assembly is obtained.

The reinforcement element may extend essentially only in an axial direction of the tubular shaft. However, additionally or alternatively the reinforcement element may extend both in an axial and circumferential direction of the tubular shaft, such as in a helical shape. By such an arrangement, the reinforcement element will not only provide resistance against shape deformation in the axial direction of the tube, but also in the radial direction, thereby providing an even better resistance against kinking and the like.

The reinforcement element may be arranged embedded in the tubular shaft. However, additionally or alternatively the reinforcement element may be arranged inside a lumen of the tubular shaft, and/or be arranged on an outer wall of the tubular shaft. Arrangement of the reinforcement element embedded in the tubular shaft provides the same structure and appearance of the tubular shaft both internally and externally as in conventional catheters. Arrangement of the reinforcement element e.g. around the outer surface of the catheter tube, or around the inward surface of a lumen within the catheter tube, can in many cases be arranged more easily during manufacturing.

The reinforcement element may form a non-separable, monolithic unit with the tubular shaft. Alternatively, the reinforcement element may be removably connected to the tubular shaft. In such an embodiment, the reinforcement element may be removed prior to, or during, insertion of the catheter into the urethra.

The reinforcement element preferably extends continuously along the length of the tubular shaft. However, the reinforcement element may also be divided into two or more separate sections, thereby extending discontinuously over the length of the tubular shaft.

The tubular shaft and the reinforcement element may be formed of different materials. In one embodiment, the tubular shaft is formed of a plastic material, and preferably a polymer material, and the reinforcement element is formed of a metal, such as steel, and/or a relatively rigid plastic material, such as polyester, polyether block amide (PEBAX), synthetic rubber, Kraton G 1645, thermoplastic elastomers, thermoplastic urethane rubbers, silicon rubber or nylon.

The tubular shaft is preferably coated with a hydrophilic polymer, said hydrophilic polymer exhibiting a low friction when wetted. The hydrophilic polymer may be at least one of: polyvinyl compounds, polylactames, in particular such as polyvinyl pyrrolidones, polysaccharides, in particular heparin, dextran, xanthan gum, derivatised polysaccharides, hydroxy propyl cellulose, methyl cellulose, polyurethanes, polyacrylates, polyhydroxyacrylates, polymethacrylates, polyacrylamides, polyalkylene oxides, in particular polyethylene oxides, polyvinyl alcohols, polyamides, polyacrylic acid, copolymers of the previously mentioned polymers, copolymers of vinyl compounds and acrylates or anhydrides, copolymers of vinylpyrrolidone and hydroxy ethylmethyl acrylate, cationic copolymers of polyvinyl pyrrolidone and copolymer of polymethylvinyl ether and maleinic acid anyhydride, polyactide, polyethylene glycol and copolymers thereof. Preferably, the hydrophilic polymer is polyvinyl pyrrolidone.

The hydrophilic coating preferably forms a polyurea network, and most preferably the polyurea network is arranged to form a covalent bond to active hydrogen groups in the substrate. Alternatively, the hydrophilic coating may form an ester bond or an epoxy bond to active hydrogen groups in the substrate.

According to one embodiment, coating of the substrate material may be made by a process comprising the steps of: applying sequentially to the surface of the substrate first a solution comprising between 0.05 to 40% (weight to volume) of an isocyanate compound and thereafter a solution containing between 0.5 and 50% (weight to volume) of polyvinylpyrrolidone and curing at an elevated temperature.

However, other hydrophilic coatings are also feasible, such as a coating comprising hydrophilic polymers cross-linked directly to the substrate. The crosslinking may be effected by means of irradiation, e.g. by electron beams or UV light.

The tubular shaft substrate may be formed of a large variety of different substrate materials. However, preferably the substrate is made of a polymer material. The substrate may e.g. comprise at least one of: polyurethanes, latex rubbers, silicon rubbers, other rubbers, polyvinylchloride (PVC), other vinyl polymers, polyesters, polyacrylates, polyamides, polyolefines, thermoplastic elastomers, styrene block copolymers (SEBS), or polyether block amide (PEBA).

The assembly may further comprise a wetting fluid for activation of said hydrophilic surface coating, said wetting fluid being accommodated by said package. In one embodiment, the wetting fluid is arranged in a wetting fluid compartment keeping the wetting fluid separated from said hydrophilic surface coating of said catheter during accommodation in said receptacle, wherein said wetting fluid is releasable in an activation state to be brought into contact with said hydrophilic surface coating prior to intended use. The wetting fluid compartment may be a wetting fluid container arranged within said receptacle. However, the wetting fluid may also be arranged in wetting contact with said hydrophilic surface coating, for preservation of said hydrophilic surface coating in a wetted state during accommodation in said receptacle and provision of a ready-to-use catheter assembly.

The receptacle may form a circular compartment. However, alternative shapes are also feasible. For example, the receptacle may form a rectangular compartment.

In one embodiment, the receptacle comprises an open ended compartment, such as a trough, and a removable lid.

The receptacle may be formed of two sheets of foil material being joined along the edges of the foils, thereby forming a closed compartment therein for housing said catheter. One of said sheets may further be provided with a re-sealable opening, and preferably a peel opening.

The maximum length of said catheter assembly is preferably less than half a length of said catheter, and preferably about less than a third of said catheter length. The length of said catheter preferably exceeds 200 mm, and even more preferably exceeds 300 mm.

These and other aspects of the invention will be apparent from and elucidated with reference to the embodiments described hereinafter.

### Brief description of the drawings

By way of example, embodiments of the invention will now be described with reference to the accompanying drawings in which:
Fig. 1 illustrates a perspective view and a cross-sectional view of an embodiment of a reinforced catheter according to an embodiment of the invention;
Fig. 2 illustrates a perspective view and a cross-sectional view of an embodiment of a reinforced catheter according to another embodiment of the invention;
Fig. 3 illustrates a perspective view and a cross-sectional view of an embodiment of a reinforced catheter according to still another embodiment of the invention;
Fig. 4 illustrates a perspective view and a cross-sectional view of an embodiment of a reinforced catheter according to still another embodiment of the invention;
Fig. 5 illustrates a perspective view of how to determine shape deformation of a catheter;
Fig. 6a illustrates an exploded perspective view of a urinary catheter assembly in accordance with one embodiment of the present invention, and Fig. 6b illustrates the same urinary catheter assembly in a cross-sectional view;
Fig. 7a illustrates an exploded perspective view of a urinary catheter assembly in accordance with another embodiment of the present invention, and Fig. 7b illustrates the same urinary catheter assembly in a cross-sectional view; and
Fig. 8a illustrates an exploded perspective view of a urinary catheter assembly in accordance with another embodiment of the present invention, and Fig. 8b illustrates the same urinary catheter assembly in a cross-sectional view.

### Description of preferred embodiments

In the following detailed description preferred embodiments of the invention will be described. However, it is to be understood that features of the different embodiments are exchangeable between the embodiments and may be combined in different ways, unless anything else is specifically indicated. It may also be noted that, for the sake of clarity, the dimensions of certain components illustrated in the drawings may differ from the corresponding dimensions in real-life implementations. Even though in the following description, numerous specific details are set forth to provide a more thorough understanding of the present invention, it will be apparent to one skilled in the art that the present invention may be practiced without these specific details. In other instances, well known constructions or functions are not described in detail, so as not to obscure the present invention.

The following discussion is in particular concerned with hydrophilic urinary catheters for intermittent use. However, the invention can also be used in relation to other types of urinary catheters.

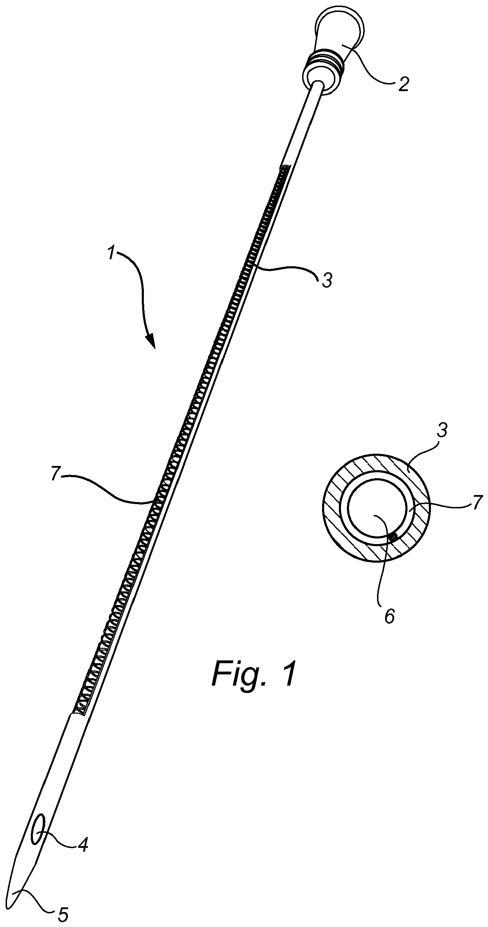

A catheter 1 as illustrated in Fig. 1, comprises a flared rearward portion 2 and an elongate shaft or tube 3 projecting forwardly from the rearward portion 2. An open-ended internal lumen 6 extends from the rear end of the rearward portion 2 to a drainage aperture 4 in a rounded tip 5 of the elongate tube 3. The rearward portion 2 may function as a connector of the catheter 1, being connectable to other devices, such as a urine collection bag, a drainage tube or the like.

The catheter is further provided with a reinforcement element 7. In this embodiment, the reinforcement element 7 is arranged as a helical element, arranged within the lumen of the tubular shaft, and abutting the interior surface of the tubular shaft.

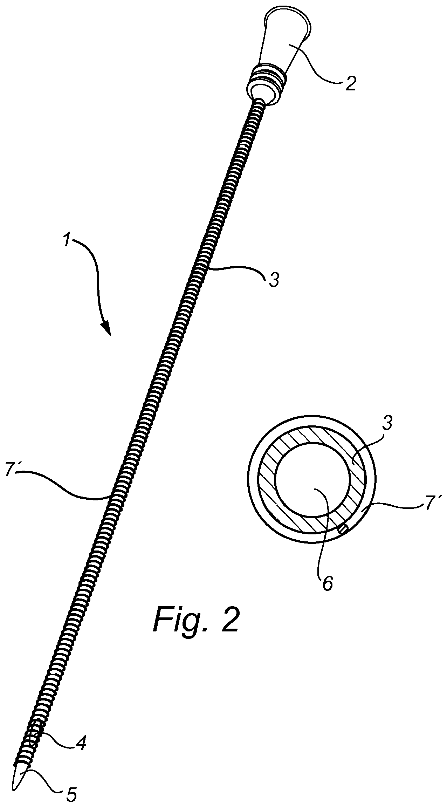

However, the reinforcement element may also be arranged in other ways in the tubular shaft. Some alternative arrangements are e.g. illustrated in Figs. 2-4.

In one alternative embodiment, as illustrated in Fig. 2, the reinforcement element 7' is instead arranged as a helical element, arranged around the exterior surface of the tubular shaft. In this embodiment, the reinforcement element may also be removable, and may e.g. be removable prior to or during insertion of the catheter into the urethra.

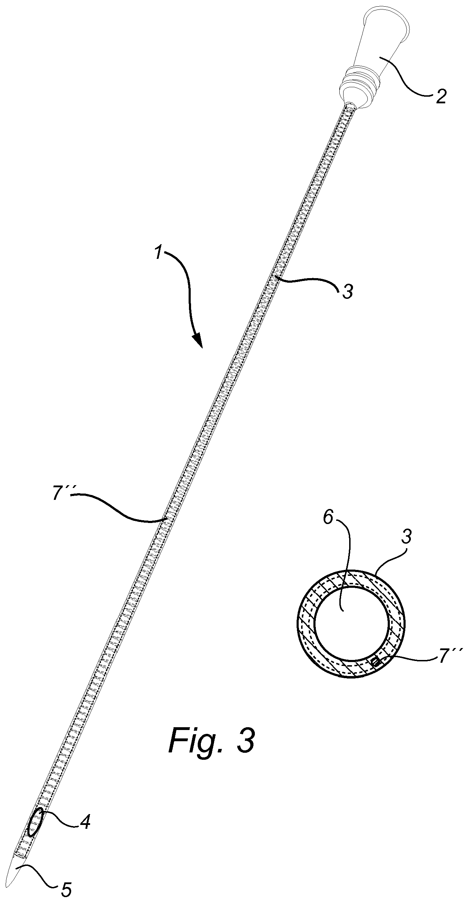

In another alternative embodiment, as illustrated in Fig. 3, the reinforcement element 7" is instead arranged as a helical element, monolithically arranged within the tubular shaft.

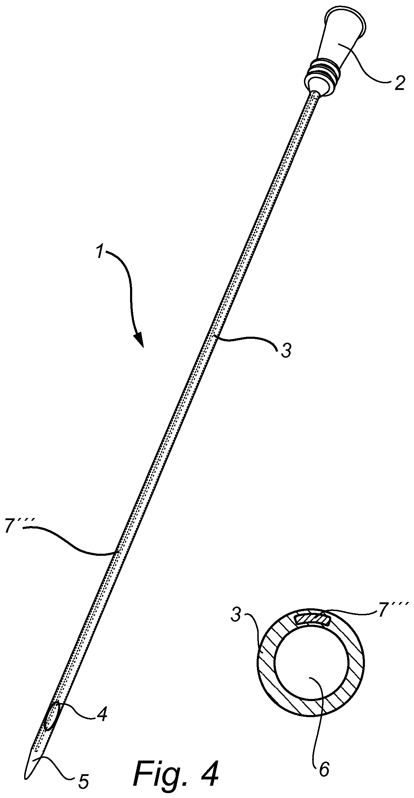

In another alternative embodiment, as illustrated in Fig. 4, the reinforcement element 7"' is instead arranged as a longitudinally extending element, monolithically arranged within the tubular shaft.

At least a part of the elongate tube 3 forms an insertable length to be inserted through a body opening of the user, such as the urethra in case of a urinary catheter. By insertable length is normally meant that length of the elongate tube 2 which is coated with a hydrophilic material, for example PVP, or which is made of hydrophilic material, and which is insertable into the urethra of the patient. Typically, this will be 80-140 mm for a female patient and 200-350 mm for a male patient.

The elongate shaft/tube of the catheter is made of a substrate material. The substrates may be made from any polymer material, which are well-known in the technical field and to which the said hydrophilic polymers adhere, such as polyurethanes, latex rubbers, other rubbers, polyvinylchloride, polyether block amide (PEBA), other vinyl polymers, polyesters and polyacrylates. However, preferably the substrate is made of a polymer blend comprising a polyolefin and a composition having molecules with active hydrogen groups, and preferably a composition having molecules with active hydrogen groups. The polyolefin can comprise at least one polymer selected from the group: polyethene, polypropene, and styrene block copolymer (SEBS). Other thermoplastic elastomers may also be used. The composition having molecules with active hydrogen groups can be a polymer having active hydrogen groups bound to the polymer via nitrogen, such as polyamide or polyurethane, or via oxygen, such as polyvinyl alcohol and poly acrylic acid.

The catheter is further coated, as have been discussed in the foregoing. In particular, for catheters, it is preferred to coat the outer surface, at least of the insertable part, with a hydrophilic coating. Many different types of well-known hydrophilic surfaces can be used. For example, the catheter may be provided with a hydrophilic coating wherein the hydrophilic polymer coating comprises material selected from polyvinyl compounds, polysaccharides, polyurethanes, polyacrylates or copolymers of vinyl compounds and acrylates or anhydrides, especially polyethyleneoxide, polyvinyl-pyrrolidone, heparin, dextran, xanthan gum, polyvinyl alcohol, hydroxy propyl cellulose, methyl cellulose, copolymer of vinylpyrrolidone and hydroxy ethylmethyl acrylate or copolymer of polymethylvinyl ether and maleinic acid anyhydride. The preferred hydrophilic polymer is polyvinylpyrrolidone, polyactide, polyethylene glycol and copolymers thereof.

The reinforcement element can e.g. be made as wires or filaments of plastic material such as polyester, nylon, Kevlar, polythene or polypropylene, or by metal, such as steel, or any combination of such materials.

The reinforcement elements preferably have a circular or rectangular cross-section, and may e.g. have a maximum cross-sectional dimension in the range of 0.01-3.0 mm, and preferably in the range of 0.1-2.0 mm, and most preferably in the range of 0.5-1.5 mm.

The reinforcement elements may be arranged to extend helically, linearly in an axial direction, or in other arrangements. Further, the number of reinforcement elements in each catheter may be one, two, three or more. In case more than one reinforcement element is used, the reinforcement elements may be arranged in the same or different arrangements, such as being arranged in parallel linear paths, parallel helical paths, or cross spiraled helical paths.

The reinforcement elements are preferably arranged with a straight, linear resting position as a default. However, the reinforcement elements may also have a curved resting position, so that the reinforced catheters are biased, and naturally curved in a direction opposite the curling direction used for the subsequent storage.

The tubular substrate of the catheter can e.g. be made by extrusion or injection molding. The reinforcement element(s) can be incorporated with the tubular shaft during manufacturing, i.e. during injection molding or extrusion. For example, reinforcement element(s) may be added in the mold cavity before injecting liquid substrate material into the mold. Alternatively, the reinforcement element(s) may be arranged on or in the tubular shaft after production of the same.

For injection molding, a liquid substrate material to be injected is preferably used, such as a thermoplastic material. Suitable thermoplastic materials may be materials such as polyurethane, polyvinyl chloride, polyethylene and other thermoformable materials. The use of thermoplastic materials means that the construction or the shape of the catheter may be partly or fully provided by treating the catheter or the catheter material with heat, such as melting or by solidifying the material by cooling.

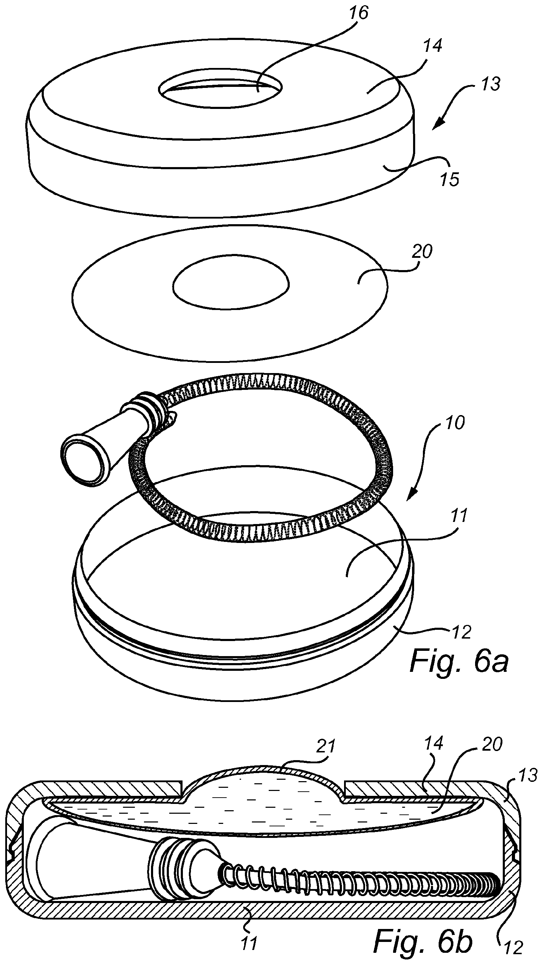

In Figs. 6-8, different embodiments of receptacles arranged to accommodate the catheter in a rolled/curled state are illustrated. In these embodiments, the receptacles are all illustrated as having a circular shape. However, other shapes are also feasible, such as a rectangular shape, an oval shape, and the like. In such embodiments, the receptacle may be made of a relatively flexible material, forming a rectangular bag or pouch, but may also be made of more rigid materials.

In the embodiment illustrated in Fig. 6, the receptacle has a pill box shape, and comprises a lower part 10, having a bottom 11 and a rim forming a circular wall 12. The open end is closed by an upper lid 13. The lid may e.g. be formed with an upper surface 14, surrounded by a rim 15, which is arranged to at least partly overlap the circular wall 12 of the lower part. In this embodiment, the lid is preferably connected to the lower part by means of a snap lock arrangement, thereby enabling reclosing of the receptacle after use. The lid is here provided as a completely removable lid. However, the lid may alternatively be hingedly connected to the bottom part.

In this embodiment, the catheter is arranged in one coil/spiral turn, thereby extending in slightly more than a full circle.

The catheter assembly is further preferably provided with a wetting fluid. The wetting fluid may be arranged separate from the catheter, in a wetting fluid container 20, such as a pouch or a sachet. The wetting fluid container is openable by means of e.g. exerting a pressure to the container, whereby the wetting fluid is released into the package, thereby wetting the hydrophilic surface of the catheter. However, alternatively the wetting fluid may be arranged in direct contact with the catheter and the hydrophilic surface during storage, thereby providing an immediately ready-to-use catheter assembly.

The wetting fluid container may be provided as a pouch or sachet loosely arranged within the receptacle. However, preferably the wetting fluid compartment is fixedly connected to either the lid or the bottom part, or both. In the example of Fig. 6, the wetting fluid compartment 20 is arranged as a pouch fixedly connected to the inside of the lid. Further, the lid is provided with an opening 16 in the upper surface, through it is possible to exert a force on the wetting fluid compartment. The wetting fluid compartment may further be arranged to bulge out with a protruding bulge 21 from this opening 16. Further, at least one point, line or area of the downwardly facing lower part of the wetting fluid compartment is provided with a weakening, such as a line or point of weakening, an area with lower wall thickness or the like. Thus, by exerting a force on the wetting fluid compartment through the opening, e.g. by pressing the bulge 21 with the thumb or any other finger into the opening, the wetting fluid compartment breaks open in the lower part, leading to a discharge of the wetting fluid into the receptacle, and consequently a wetting of the catheter accommodated therein.

The wetting fluid is preferably an aqueous liquid, such as water or saline. Such wetting fluid containers and wetting fluids are per se well known in the art.

The receptacle is in this embodiment made of a relatively rigid plastic material, thereby making the parts self-supporting, whereas the wetting fluid compartment can be made of a relatively flexible plastic or metal material. For example, the receptacle can be made of polymer materials such as polyethene, polypropylene, polyamide, PET, or the receptacle can be made from a laminate of such polymer materials and/or aluminum, aluminum oxide, or oriented polypropylene (OPP). The flexible material used for wetting fluid pouch is preferably a flexible material which provides a moisture barrier or low water vapor transmission. The flexible material may e.g. comprise or consist of one or several of aluminum, aluminum oxide, silicone oxide, metallocene polyvinylidene chloride (PVdC) and poly(ethylene-vinylacetate) (EVA). For example, the flexible material can be made as coextruded polyolefines with polyamides, polyethylene terephtalate (PET), including barrier resins such as polyvinylidene chloride (PVdC) or poly(ethylene-vinylacetate) (EVA). However, other materials exhibiting similar properties are also feasible to use.

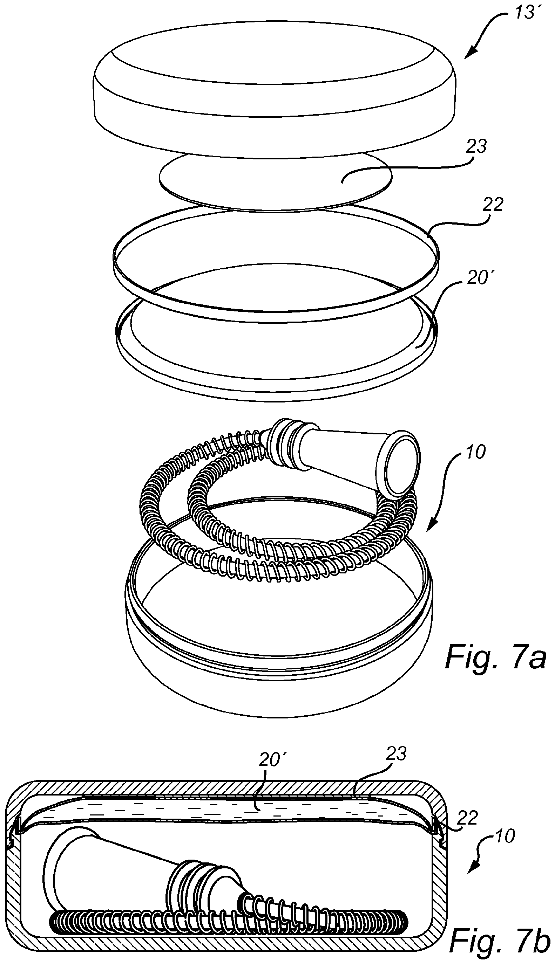

The embodiment of Fig. 7 shows a similar type of catheter assembly. Here the catheter is arranged in two coils, i.e. extending over about two full circles, to provide two mutually superposed spiral turns. However, more than two coils are also feasible. In this embodiment, a different type of release arrangement for the wetting fluid is provided. In this embodiment, the lid is not provided with an opening. Instead, the wetting fluid compartment 20' is attached to the bottom part of the receptacle by means of a locking ring 22 or the like. Further, the upper part of the wetting fluid compartment 20' is adhered to the inside of the lid by means of an adhesive layer 23 or the like. Hereby, the wetting fluid compartment is connected both to the bottom part and the lid of the receptacle. By providing a relative movement between the lower part and the lid, e.g. by rotating the lid or by opening the lid, the walls of the wetting fluid compartment are pulled apart, leading to breaking of the wall in one or several places, and thereby a release of the wetting fluid.

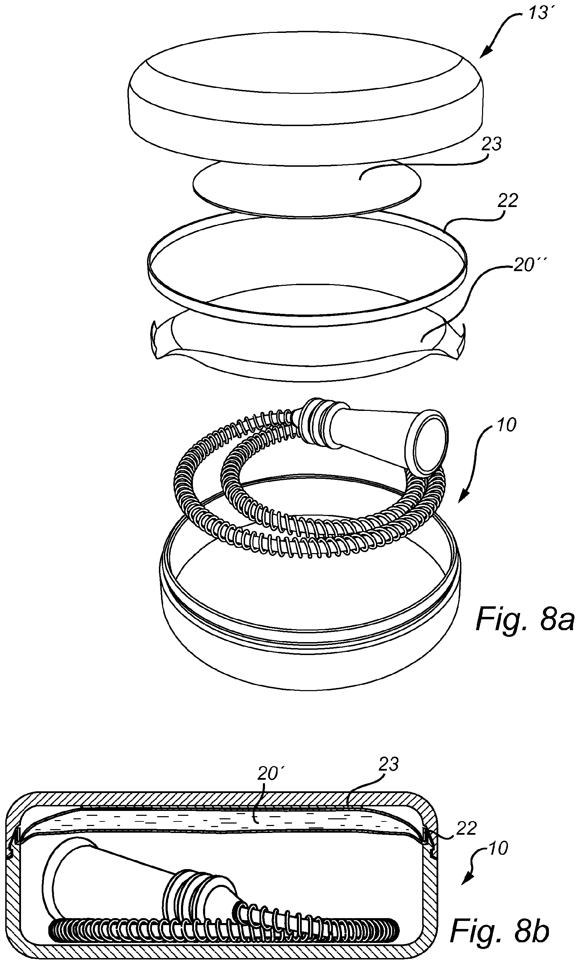

A similar arrangement is illustrated in Fig. 8, but here the wetting fluid compartment 20" is only connected to the lower part at a few locations, here at two oppositely arranged positions, instead of around the whole circumference, as in Fig. 7.

However, many other alternative arrangements for providing a supply of wetting fluid in the receptacle, and for enabling a release of the wetting fluid when needed, are feasible, as would be apparent for the skilled addressee.

Further, even if the bottom part is made of a relatively rigid material, the lid need not. Instead, the lid may e.g. be made as a peelable membrane arranged over an opening of the lower part.

Further, the lower part need also not be made of a relatively rigid material, as illustrated in the above-discussed embodiments. Instead, the package may be formed of flexible sheet material, such as a first sheet material and a second sheet material, connected around the edges to form an inner cavity housing the catheter and the wetting fluid. The first and second sheet materials are then preferably connected around the edges by means of welding, forming a welded edge joint. Preferably, the first and second sheet materials comprise laminated sheets, having a weldable inner layer and a protective outer layer. However, one or both of the sheets may alternatively be non-laminated, and be made of e.g. extruded or co-extruded material. The sheet materials are preferably of a flexible plastics material. For example, the sheets can be made of polymer materials such as polyethene, polypropylene, polyamide, and PET, or the receptacle can be made from a laminate of such polymer materials and/or aluminum, aluminum oxide, or oriented polypropylene (OPP). One or both of the sheets may also be deep-drawn into a through shape or the like.

For opening such a sheet package, a tear opening or peel opening, separating the sheets or tearing one or both of the sheets apart, are feasible. However, the sheet package may alternatively be provided with a resealable opening. Such a resealable opening may e.g. be formed by forming a wholly or partially cut through non-closed loop in one of the sheets, and to add a further opening sheet on top of this loop. Attachment of the sheet may e.g. be made by adhesive.

Although the wetting fluid in the described embodiments has been arranged separated from the catheter, in a wetting fluid container, it is also possible to arrange the wetting fluid in direct contact with the catheter, thereby always maintaining a ready-to-use state.

Upon use, the catheter is then wetted by a wetting fluid, whereby the hydrophilic surface becomes slippery and easy to insert into e.g. the urethra of the patient, i.e. to provide a low-friction character of the surface. After wetting, the catheter is extracted from the receptacle, and used in a conventional way. After use, the catheter may be returned to the receptacle, which is subsequently closed, which make disposal of the catheter assembly in a discreet manner even simpler.

In an experimental study, conventional LoFric® catheters made by Wellspect Healthcare, part of Dentsply IH AB, were compared to the same type of catheters retrofitted with reinforcement elements. The catheters were hydrophilic urinary catheters of two sizes: Ch 12 and Ch 14. The reinforcement elements were coil springs of steel arranged within the lumen of the catheters. The catheters were placed in boxes of a circular, having a diameter of 6.6 cm, and a height of 2.2 cm, and with a removable lid. The catheters were of about 25 cm length, and were consequently arranged in more than one full turn/coil in the receptacles. The catheter assemblies were then subjected to an accelerated aging procedure, simulating aging of 1 year storage and 3.5 year storage, respectively.

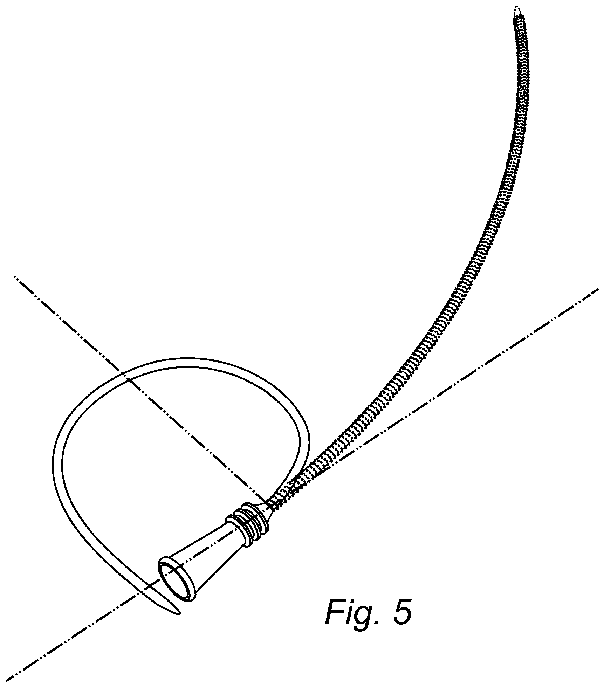

After the aging, the catheters were removed from the receptacle, and the shape and linearity of the catheters were studied. Specifically, the angle between a line extending between the connector and the tip of the catheter and a line extending in the elongation of the axial direction of the connector was determined, as is schematically illustrated in Fig. 5. Here, the angle for the exemplary catheter drawn in solid lines is more than 180° (the catheter is still within a full spiral turn), whereas the schematically illustrated reinforced catheter, drawn in dashed lines, has an angle of about 30°. In the experimental study, the angles were determined at specific times after removal of the catheter from the receptacle, viz. at 0 minutes, 0.5 minute, 1 minute, 2 minutes and 5 minutes. At least five samples of each size and type (reinforced or not reinforced) were tested.

The results of this study are presented in the table below, where:
- Ex 1 = Catheter of size Ch 12, with reinforcement, and after 1 year of aging.
- Ex 2 = Catheter of size Ch 14, with reinforcement, and after 1 year of aging.
- Ex 3 = Catheter of size Ch 12, with reinforcement, and after 3.5 years of aging.
- Ex 4 = Catheter of size Ch 14, with reinforcement, and after 3.5 years of aging.
- Comp. Ex A = Catheter of size Ch 12, without reinforcement, and after 3.5 year of aging.
- Comp. Ex B = Catheter of size Ch 14, with reinforcement, and after 3.5 years of aging.

The angles below are the average angles of the studied samples.

| **Catheter** | **0 min** | **0.5 min** | **1 min** | **2 min** | **5 min** |
|---|---|---|---|---|---|
| Ex. 1 | 61 | 55 | 50 | 33 | 26 |
| Ex. 2 | 124 | 97 | 87 | 80 | 76 |
| Ex. 3 | 64 | 41 | 37 | 32 | 29 |
| Ex.4 | 126 | 105 | 88 | 81 | 68 |
| Comp. Ex. A | 180 | 180 | 180 | 180 | 180 |
| Comp. Ex. B | 180 | 180 | 180 | 180 | 180 |

From the following it may be noted that the reinforcement elements provide a very efficient remedy to the shape deformation that otherwise occurs in the catheters after long time storage when stored in a curled state. The comparative examples, without reinforcement elements, remain in a completely curled state after removal from the receptacle for the full measured period of 5 minutes. Such a completely curled catheter would be very difficult to handle efficiently for an ordinary user. In comparison, the reinforced catheters of Ex. 1-4 all reassume a much more linear shape after removal from the receptacles. Further, this process progressed relatively quickly, with much improvement occurring in a relatively short time. In less than 1 minute, all the catheters according to the invention showed an angle of less than 90°, which is fully acceptable for an ordinary user. Further, the catheters with Ch12 in accordance with the invention and aged for 1 year and 3,5 years, respectively (Ex. 1 and 3), were immediately possible to use, immediately reassuming an angle of less than 65°.

In another line of experiments, the stiffnesses of the catheters were determined by experiments as outlined in "Stiffness of Central Venous Catheters", Acta Anaesthesiol Scand 1983: 27: 153-157, by Stenqvist et. al. It was found that the reinforced catheters, as discussed above, provide much improved resistance against deterioration due to sterilization by means of irradiation. It was also found that when the stiffness, when measured as a force to displace an extending portion of a catheter shaft, was at least 2 times higher for the reinforced catheters compared to non-reinforced catheters, a very good uncurling of the catheters having been stored in a curled condition was obtained. This was improved even further when the stiffness was increased even further, to 3 times, and even further when increased to 3.5 times.

## Claims

1. A urinary catheter assembly comprising:
a urinary catheter (1) having a tubular shaft (3) extending between a insertable end and a discharge end,; and
a receptacle arranged to accommodate and maintain the urinary catheter in a curled storage state, in which the tubular shaft (3) of the catheter is arranged in at least one coil;
**characterized in that** said tubular shaft (3) is provided with an elastic reinforcement element (7; 7'; 7"; 7"') having an extension in the axial direction of the tubular shaft, and **in that** said elastic reinforcement element (7; 7'; 7"; 7"') is arranged to provide a force to uncurl the catheter when removed from said receptacle.

2. The urinary catheter assembly of claim 1, wherein the receptacle is arranged to accommodate and maintain the urinary catheter (1) in a curled storage state, in which the tubular shaft (3) of the catheter is arranged in at least two coils, and preferably at least three coils.

3. The urinary catheter assembly of claim 1 or 2, wherein the reinforcement element (7"') extends essentially only in an axial direction of the tubular shaft (3).

4. The urinary catheter assembly of claim 1 or 2, wherein the reinforcement element (7; 7'; 7") extends both in an axial and circumferential direction of the tubular shaft.

5. The urinary catheter assembly of claim 4, wherein the reinforcement element (7; 7'; 7") is helically shaped.

6. The urinary catheter assembly of any one of the preceding claims, wherein the reinforcement element (7"; 7"') is arranged embedded in the tubular shaft (3).

7. The urinary catheter assembly of any one of the claims 1-5, wherein the reinforcement element (7) is arranged inside a lumen of the tubular shaft (3).

8. The urinary catheter assembly of any one of the claims 1-5, wherein the reinforcement element (7') is arranged on an outer wall of the tubular shaft (3).

9. The urinary catheter assembly of any one of the preceding claims, wherein the reinforcement element (7; 7"; 7"') forms a non-separable, monolithic unit with the tubular shaft (3).

10. The urinary catheter assembly of any one of the claims 1-8, wherein the reinforcement element (7') is removably connected to the tubular shaft (3).

11. The urinary catheter assembly of any one of the preceding claims, wherein the tubular shaft (3) and the reinforcement element (7; 7'; 7"; 7"') are formed of different materials.

12. The urinary catheter assembly of claim 11, wherein the tubular shaft (3) is formed of a plastic material, and preferably a polymer material, and wherein the reinforcement element (7; 7'; 7"; 7"') is formed of a metal, such as steel, and/or a relatively rigid plastic material, such as polyester or nylon.

13. The urinary catheter assembly of any one of the preceding claims, wherein the tubular shaft (3) is coated with a hydrophilic polymer, said hydrophilic polymer exhibiting a low friction when wetted.

14. The urinary catheter assembly of claim 13, wherein the assembly further comprises a wetting fluid for activation of said hydrophilic surface coating, said wetting fluid being accommodated by said package.

15. The urinary catheter assembly of any one of the preceding claims, wherein the receptacle forms a circular compartment.

16. The urinary catheter assembly of any one of the preceding claims, wherein the receptacle comprises an open-ended compartment and a removable lid (13; 13').

17. The urinary catheter assembly of any one of the claims 1-15, wherein the receptacle is formed of two sheets of foil material being joined along the edges of the foils, thereby forming a closed compartment therein for housing said catheter.

## Patentansprüche

1. Harnkathetereinheit, umfassend:
einen Harnkatheter (1), der einen rohrförmigen Schaft (3) hat, der sich zwischen einem einführbaren Ende und einem Austrittsende erstreckt; und
einen Behälter, der zum Aufnehmen und Bewahren des Harnkatheters in einem eingerollten Lagerzustand ausgelegt ist, in dem der rohrförmige Schaft (3) des Katheters in mindestens einer Windung angeordnet ist;
**dadurch gekennzeichnet, dass** der rohrförmige Schaft (3) mit einem elastischen Verstärkungselement (7; 7'; 7"; 7"') versehen ist, das eine Ausdehnung in der axialen Richtung des rohrförmigen Schaftes hat, und
dadurch, dass das elastische Verstärkungselement (7; 7'; 7"; 7"') dafür ausgelegt ist, eine Kraft zum Strecken des Katheters auszuüben, wenn er aus dem Behälter entnommen wird.

2. Harnkathetereinheit nach Anspruch 1, wobei der Behälter dafür ausgelegt ist, den Harnkatheter (1) in einem aufgerollten Lagerzustand aufzunehmen und zu halten, wobei der rohrförmige Schaft (3) des Katheters in mindestens zwei Windungen und vorzugsweise drei Windungen angeordnet ist.

3. Harnkathetereinheit nach Anspruch 1 oder 2, wobei das Verstärkungselement (7"') sich im Wesentlichen nur in einer axialen Richtung des rohrförmigen Schaftes (3) erstreckt.

4. Harnkathetereinheit nach Anspruch 1 oder 2, wobei das Verstärkungselement (7; 7'; 7") sich sowohl in einer axialen als auch in einer Umfangsrichtung des rohrförmigen Schaftes erstreckt.

5. Harnkathetereinheit nach Anspruch 4, wobei das Verstärkungselement (7; 7'; 7") schraubenförmig ist.

6. Harnkathetereinheit nach einem der vorherigen Ansprüche, wobei das Verstärkungselement (7"; 7"') in den rohrförmigen Schaft (3) eingebettet ist.

7. Harnkathetereinheit nach einem der Ansprüche 1-5, wobei das Verstärkungselement (7) in einem Lumen des rohrförmigen Schaftes (3) angeordnet ist.

8. Harnkathetereinheit nach einem der Ansprüche 1-5, wobei das Verstärkungselement (7) auf einer Außenwand des rohrförmigen Schaftes (3) angeordnet ist.

9. Harnkathetereinheit nach einem der vorherigen Ansprüche, wobei das Verstärkungselement (7; 7"; 7"') eine untrennbare, monolithische Einheit mit dem rohrförmigen Schaft (3) bildet.

10. Harnkathetereinheit nach einem der Ansprüche 1-8, wobei das Verstärkungselement (7') lösbar mit dem rohrförmigen Schaft (3) verbunden ist.

11. Harnkathetereinheit nach einem der vorherigen Ansprüche, wobei der rohrförmige Schaft (3) und das Verstärkungselement (7; 7'; 7"; 7"') aus verschiedenen Materialien gebildet sind.

12. Harnkathetereinheit nach Anspruch 11, wobei der rohrförmige Schaft (3) aus einem Plastikmaterial gebildet ist und vorzugsweise aus einem Polymermaterial, und wobei das Verstärkungselement (7; 7'; 7"; 7"') aus einem Metall gebildet ist, wie zum Beispiel Stahl, und/oder einem relativ starren Plastikmaterial, wie zum Beispiel Polyester oder Nylon.

13. Harnkathetereinheit nach einem der vorherigen Ansprüche, wobei der rohrförmige Schaft (3) mit einem hydrophilen Polymer beschichtet ist, wobei das hydrophile Polymer eine geringe Reibung aufweist, wenn es benetzt ist.

14. Harnkathetereinheit nach Anspruch 13, wobei die Einheit ferner ein Benetzungsfluid zum Aktivieren der hydrophilen Oberflächenbeschichtung umfasst, wobei das Benetzungsfluid von der Packung aufgenommen wird.

15. Harnkathetereinheit nach einem der vorherigen Ansprüche, wobei der Behälter einen kreisförmigen Raum bildet.

16. Harnkathetereinheit nach einem der vorherigen Ansprüche, wobei der Behälter einen offenen Raum und einem abnehmbaren Deckel (13; 13') umfasst.

17. Harnkathetereinheit nach einem der Ansprüche 1-15, wobei der Behälter aus zwei Bögen von Folienmaterial gebildet wird, die entlang der Kanten der Folien verbunden werden, wodurch ein geschlossener Raum darin zum Aufnehmen des Katheters gebildet wird.

## Revendications

1. Ensemble de cathéter urinaire comprenant :
un cathéter urinaire (1) doté d'un arbre tubulaire (3) s'étendant entre une extrémité insérable et une extrémité de décharge ; et
un réceptacle conçu pour abriter et maintenir le cathéter urinaire dans un état de stockage enroulé dans lequel l'arbre tubulaire (3) est disposé en au moins une boucle ;
**caractérisé en ce que** ledit arbre tubulaire (3) est pourvu d'un élément de renfort élastique (7 ; 7' ; 7" ; 7'") ayant une extension dans le sens axial de l'arbre tubulaire, et **en ce que** ledit élément de renfort élastique (7 ; 7' ; 7" ; 7'") est conçu pour fournir une force pour dérouler le cathéter lorsqu'il est retiré dudit réceptacle.

2. Ensemble de cathéter urinaire selon la revendication 1, dans lequel le réceptacle est conçu pour abriter et maintenir le cathéter urinaire (1) dans un état de stockage enroulé dans lequel l'arbre tubulaire (3) du cathéter est disposé en au moins deux boucles et de préférence au moins trois boucles.

3. Ensemble de cathéter urinaire selon la revendication 1 ou 2, dans lequel l'élément de renfort (7'") s'étend essentiellement seulement dans un sens axial de l'arbre tubulaire (3).

4. Ensemble de cathéter urinaire selon la revendication 1 ou 2, dans lequel l'élément de renfort (7 ; 7' ; 7") s'étend à la fois dans le sens axial et le sens circonférentiel de l'arbre tubulaire.

5. Ensemble de cathéter urinaire selon la revendication 4, dans lequel l'élément de renfort (7 ; 7' ; 7") est de forme hélicoïdale.

6. Ensemble de cathéter urinaire selon l'une quelconque des revendications précédentes, dans lequel l'élément de renfort (7" ; 7'") est disposé encastré dans l'arbre tubulaire (3).

7. Ensemble de cathéter urinaire selon l'une quelconque des revendications 1 à 5, dans lequel l'élément de renfort (7) est disposé à l'intérieur d'un lumen de l'arbre tubulaire (3).

8. Ensemble de cathéter urinaire selon l'une quelconque des revendications 1 à 5, dans lequel l'élément de renfort (7') est disposé sur une paroi extérieure de l'arbre tubulaire (3).

9. Ensemble de cathéter urinaire selon l'une quelconque des revendications précédentes, dans lequel l'élément de renfort (7 ; 7" ; 7'") forme une unité monolithique non séparable avec l'arbre tubulaire (3).

10. Ensemble de cathéter urinaire selon l'une quelconque des revendications 1 à 8, dans lequel l'élément de renfort (7') est connecté de manière amovible à l'arbre tubulaire (3).

11. Ensemble de cathéter urinaire selon l'une quelconque des revendications précédentes, dans lequel l'arbre tubulaire (3) et l'élément de renfort (7 ; 7' ; 7" ; 7'") sont composés de différents matériaux.

12. Ensemble de cathéter urinaire selon la revendication 11, dans lequel l'arbre tubulaire (3) est composé d'une matière plastique, et de préférence d'une matière polymérique, et l'élément de renfort (7 ; 7' ; 7" ; 7'") est composé d'un métal, tel que de l'acier, et/ou une matière plastique relativement rigide, telle que du polyester ou du nylon.

13. Ensemble de cathéter urinaire selon l'une quelconque des revendications précédentes, dans lequel l'arbre tubulaire (3) est revêtu d'un polymère hydrophile, ledit polymère hydrophile présentant une faible friction quand il est mouillé.

14. Ensemble de cathéter urinaire selon la revendication 13, dans lequel l'ensemble comprend en outre un fluide de mouillage pour l'activation dudit revêtement de surface hydrophile, ledit fluide de mouillage étant logé dans ledit emballage.

15. Ensemble de cathéter urinaire selon l'une quelconque des revendications précédentes, dans lequel le réceptacle forme un compartiment circulaire.

16. Ensemble de cathéter urinaire selon l'une quelconque des revendications précédentes, dans lequel le réceptacle comprend un compartiment à extrémité ouverte et un couvercle amovible (13 ; 13').

17. Ensemble de cathéter urinaire selon l'une quelconque des revendications 1 à 15, dans lequel le réceptacle est formé de deux feuilles de matériau en feuille qui sont jointes le long des bords des feuilles, en formant ainsi un compartiment fermé à l'intérieur pour loger ledit cathéter.
